# EUROPEAN PATENT APPLICATION

(11) **EP 2 606 917 A1**
(43) Date of publication of application: **26.06.2013**
(21) Application number: 11009992.6
(22) Date of filing: 20.12.2011
(51) Int. Cl.: A61L 27/30, A61L 27/54, A61L 27/56

(54) **Porous scaffold with carbon-based nanoparticles**

(71) Applicant: Steinmüller-Nethl, Doris, 6074 Aldrans (AT); Fraunhofer-Gesellschaft zur Förderung der angewandten Forschung e.V., 80686 München (DE)
(72) Inventor: Steinmüller-Nethl, Doris, Dr., 6074 Aldrans (AT); Walles, Heike, Prof., 71063 Sindelfingen (DE)
(74) Representative: Wohlfahrt, Jan Günther

(57) **Abstract**

The present invention refers to biocompatible porous scaffolds, especially bone regeneration scaffolds, comprising carbon-based nanoparticles, especially diamond nanoparticles, methods for the production of such biocompatible porous scaffolds, the use of such biocompatible porous scaffolds and methods for treating bone defects by inserting such biocompatible porous scaffolds comprising carbon-based nanoparticles into the bone defect.

## Description

The present invention refers to biocompatible porous scaffolds, especially bone regeneration scaffolds, comprising carbon-based nanoparticles, especially diamond nanoparticles, methods for the production of such biocompatible porous scaffolds, the use of such biocompatible porous scaffolds and methods for treating bone defects by inserting such biocompatible porous scaffolds comprising carbon-based nanoparticles into the bone defect.

Regeneration of physiological bone tissue comprises the steps of angiogenesis, formation of a callus and modification of the bone. In the regeneration process growth factors like VEGF, PDGF-BB, PIGF, BMPs and bFGF are very important. However, the efficiency of bone regeneration is not sufficient if the bone defect is too large, for example larger than 1 cm. Furthermore, healing is also affected by the age of the patient or by factors like diabetes or radiation therapy. All these factors can lead to an insufficient vascular repairing and accordingly to hypoxia resulting in reduced bone formation, especially in bigger bone defects.

The best mode according to the state of the art for the therapy of the bone defects under such insufficient conditions is a transplantation of autologous bone. However, the use of autologous bone grafting leads to other problems for the patient like requirement of an additional surgical site. Allograft bone or xenograft bone can lead to un-wished side reactions or infections.

The possibility of treating bone defects with alloplastic material has been demonstrated in the past.

There are several different kinds of alloplastic bone substitute materials on the market. Improved biocompatible bone graft materials are for example described in US 2010/0075904 A1, WO 2008/002682 A2, WO 2005/074614 A2 or WO 2011/109912 A1. The materials used for such bone graft substitutes are mainly biological inert materials like titan, ceramics like beta-tricalciumphosphate, PLLA bone cement, polymethylmetacrylate, polyethylene, calciumphosphate, hydroxylapatite composites or carbon composites. However, such materials lead to weak tissue scaffold interference. The bone graft substitutes of the state of the art are not suitable for large bone defects, for example in the area of the cranium, the jar bone or the face. Using such bone graft substitutes for large bone defects often leads to an insufficient recovery and vascularisation.

Great efforts have been made to develop scaffolds with biodegradable, biocompatible and cellular active properties in order to support cell function and new tissue formation in vivo as disclosed for example in Hutmacher D. W.: Scaffolds in Tissue Engineering, Bone and Cartilage; Biomaterials (2000); 21: 2529-2543, US 2010/323094 A1, WO 2009/108935 A2 or in WO 2010/146312 A1. However, the major issue has been the limitation of cell migration, nutrient supply and tissue increase with three-dimensional constructs (Botchway E. A. et al.: Tissue Engineered Bone: Measurement of Nutrient Transport in Three-Dimensional Matrices; J. Biomed. Materes. A. (2003); 67: 357-367). All these factors depend on vessel formation within the scaffold, which is up to now the major obstacle in a restoration of large bone defects with bone substitutes. Therefore, much interest has been directed towards the modification and functionalization of biomaterials/scaffolds such as functional group grafting, protein absorption and self-assembly, plasma treatment or micro-patterning in order to improve scaffold properties.

Carbon-containing layers are often used for coating implants, since such layers have high biocompatibility and, thus, reduced rejection reactions to such a great extent that partly no such reactions are provoked any longer. Moreover, carbon-containing layers have also proven to be robust and to have low friction as disclosed for example in US 6,447,295 and EP 0 302 717.

Carbon-based nanoparticles, especially diamond nanoparticles, were originally used to act as a seeding-layer for subsequent diamond film coating of the surface of tools also disclosed in AT 504006 A1.

Nanocrystalline diamond thin films have also been used as coating for polished implants made of titanium as described in WO 2006/060836 A1, Kloss et al.: "The role of oxygen termination of nanocrystalline diamond on immobilisation of BMP-2 and subsequent bone formation"; Biomaterials (2008); 29: 2433-2442 and Kloss, F. R. et al.: "In vivo Investigation on Connective Tissue Healing to Polished Surfaces with Different Surface Wettability"; Clean Oral Implants Res (2011); 7: 699-705. The thin diamond films cover the surface of the implants so that only the diamond film, but not the implant surface has contact to the surrounding tissue. The diamond layers and films are generated in methods using temperatures of above 400 °C.

The technical problem underlying the present invention is to provide improved biocompatible porous scaffolds, especially usable as tissue regeneration scaffolds, preferably bone regeneration scaffolds, for overcoming the disadvantages of scaffolds of the state of the art.

In particular, a technical problem underlying the present invention is to provide a bone regeneration scaffold leading to a better neovascularisation, especially in large bone defects.

A further technical problem underlying the present invention is to provide tissue regeneration scaffolds, especially bone regeneration scaffolds suitable to be used in large tissue defects, especially large bone defects.

A further technical problem underlying the present invention is to provide means and methods to improve the neovascularisation in tissue defects, especially bone defects.

A further technical problem underlying the present invention is to provide improved functionalized porous scaffolds, especially wherein the degree of functionalization can be regulated at different areas of the scaffold.

In particular, a technical problem underlying the present invention is to provide means and methods for an uncomplicated use of improved bone regeneration scaffolds, especially individually functionalized bone regeneration scaffolds by a physician.

The present invention solves its underlying technical problem in particular by providing the subject matter of the claims. The technical problem is solved especially by the teaching of the independent claims.

The present invention solves its underlying technical problem in particular by providing a biocompatible porous scaffold comprising a biocompatible porous scaffold material and carbon-based nanoparticles.

The present invention solves its underlying technical problem in particular by providing a biocompatible porous scaffold comprising at least one biocompatible porous scaffold material and at least one carbon-based nanoparticle.

The present invention solves its underlying technical problem in particular by providing at least one biocompatible porous scaffold comprising at least one biocompatible porous scaffold material and a plurality of carbon-based nanoparticles.

The inventors found not only that carbon-based nanoparticles are suitable to increase the surface and the reactive part of the biocompatible porous scaffold. Furthermore the inventors found that carbon-based nanoparticles are surprisingly suitable to enhance and/or control the stiffness, i.e. modulus of elasticity, of the biocompatible porous scaffold. The inventors found also that the use of carbon-based nanoparticles, especially diamond nanoparticles leads to an improved tissue scaffold interference. Furthermore the inventors found surprisingly that biomolecules, for example proteins, can be better and in an easy way bound to a biocompatible porous scaffold via carbon-based nanoparticles. Especially the inventors found that the functional groups, for example proteins, can be bound to carbon-based nanoparticles, especially diamond nanoparticles, by physisorption and that this physical adsorption binds the biomolecules strong enough to be used during tissue regeneration. The inventors found also surprisingly that the presence of the carbon-based nanoparticles in the porous scaffold leads to an improved vascularisation in the inner area of a large bone defect. Furthermore the inventors found surprisingly that the amount of carbon-based nanoparticles which is necessary to provide such beneficial effects is not toxic when used in tissue regeneration, especially present in tissue regeneration scaffolds. Furthermore the inventors found surprisingly that carbon-based nanoparticles can be bound to the biocompatible porous scaffold material in gradient, so that different areas of the scaffold have different amounts of the carbon-based nanoparticles. Also the combination of different scaffold materials and the combination of different biomodulating substances bound to the nanoparticles can further improve the scaffold system according to the present invention. This can advantageously be used to provide different amounts of at least one biomodulating substance to different areas of the tissue surrounding the tissue regeneration scaffold when used. Furthermore the inventors found surprisingly that due to the presence of carbon-based nanoparticles active biomolecules which are bound the carbon-based nanoparticles can be present in decreased amounts without a decrease of their effect. The carbon-based nanoparticles and accordingly the biomolecules can for example be present only in the pores of the scaffold.

According to a preferred embodiment of the present invention the carbon-based nanoparticles include a material selected from the group consisting of polymers, diamond, amorphous carbon, diamond-like carbon, graphite, nano-tubes, nanowires, fullerenes, pyrocarbon, glassy carbon, and mixtures thereof.

According to a preferred embodiment of the present invention the carbon-based nanoparticles have a size of at least 0,1 and at most 500 nm. According to a preferred embodiment of the present invention the carbon-based nanoparticles have a size of at least 1 and at most 250 nm. According to a preferred embodiment of the present invention the carbon-based nanoparticles have a size of at least 2 and at most 100 nm. According to a preferred embodiment of the present invention the carbon-based nanoparticles have a size of at least 2 and at most 50 nm. According to an alternative embodiment of the present invention the carbon-based nanoparticles have a size of at least 1 and at most 20 nm. According to an alternative embodiment of the present invention the carbon-based nanoparticles have a size of at least 1 and at most 10 nm. According to an alternative embodiment of the present invention the carbon-based nanoparticles have a size of at least 3 and at most 8 nm. According to an alternative embodiment of the present invention the carbon-based nanoparticles have a size of at least 4 and at most 6 nm. According to a preferred embodiment of the present invention the carbon-based nanoparticles have a size of around 5 nm. According to a preferred embodiment of the present invention the carbon-based nanoparticles have a size of around 4 nm. According to a preferred embodiment of the present invention the carbon-based nanoparticles have a size of around 4 to 5 nm.

According to a preferred embodiment of the present invention the carbon-based nanoparticles have a size of at least 0,1 nm. According to a preferred embodiment of the present invention the carbon-based nanoparticles have a size of at least 1 nm. According to a preferred embodiment of the present invention the carbon-based nanoparticles have a size of at least 2 nm. According to an alternative embodiment of the present invention the carbon-based nanoparticles have a size of at least 3 nm.

According to a preferred embodiment of the present invention the carbon-based nanoparticles have a size of at most 1000 nm. According to a preferred embodiment of the present invention the carbon-based nanoparticles have a size of at most 500 nm. According to a preferred embodiment of the present invention the carbon-based nanoparticles have a size of at most 250 nm. According to a preferred embodiment of the present invention the carbon-based nanoparticles have a size of at most 100 nm. According to a preferred embodiment of the present invention the carbon-based nanoparticles have a size of at most 50 nm. According to a preferred embodiment of the present invention the carbon-based nanoparticles have a size of at most 25 nm. According to an alternative embodiment of the present invention the carbon-based nanoparticles have a size of at most 20 nm. According to an alternative embodiment of the present invention the carbon-based nanoparticles have a size of at most 15 nm. According to an alternative embodiment of the present invention the carbon-based nanoparticles have a size of at most 10 nm. According to an alternative embodiment of the present invention the carbon-based nanoparticles have a size of at most 8 nm.

According to a preferred embodiment of the present invention the carbon-based nanoparticles are to a high degree not agglomerated. According to a preferred embodiment of the present invention the carbon-based nanoparticles are not agglomerated. A person skilled in the art knows suitable methods to avoid agglomeration as for example described in Liang Y. et al.: Deagglomeration and Surface Modification of Thermally Annealed Nano Scale Diamond; J. Colloid Interface Sci. (2011); 354: 23-30.

According to a preferred embodiment of the present invention at least one, preferably most, more preferably almost all or all of the carbon-based nanoparticles contain diamond.

According to a preferred embodiment of the present invention the carbon-based nanoparticles have a diamond nanoparticle proportion of at least 10%, at least 20%, at least 30%, at least 40%, at least 49%, at least 50%, at least 51%, at least 60%, at least 70%, at least 80%, at least 90%, at least 95%, at least 99%, at least 99,5%, and at least 99,9% (measured as % of amount of particles).

According to a preferred embodiment of the present invention at least one, preferably most, of the carbon-based nanoparticles are diamond nanoparticles.

According to a preferred embodiment of the present invention the carbon-based nanoparticles are diamond nanoparticles.

Embodiments and preferred embodiments of the present invention relating to "carbon-based nanoparticles" are also embodiments and preferred embodiments relating to "diamond nanoparticles".

According to a preferred embodiment of the present invention the diamond nanoparticles are diamond crystals. According to a preferred embodiment of the present invention the diamond nanoparticles are diamond crystals selected from the group consisting of natural diamond, polycrystalline, microcrystalline, nanocrystalline, ultrananocrystalline and monocrystalline diamond crystals.

According to a preferred embodiment of the present invention the diamond nanoparticles have a size of at least 0,1 and at most 500 nm. According to a preferred embodiment of the present invention the diamond nanoparticles have a size of at least 1 and at most 250 nm. According to a preferred embodiment of the present invention the diamond nanoparticles have a size of at least 2 and at most 100 nm. According to a preferred embodiment of the present invention the diamond nanoparticles have a size of at least 2 and at most 50 nm. According to an alternative embodiment of the present invention the diamond nanoparticles have a size of at least 1 and at most 20 nm. According to an alternative embodiment of the present invention the diamond nanoparticles have a size of at least 1 and at most 10 nm. According to an alternative embodiment of the present invention the diamond nanoparticles have a size of at least 3 and at most 8 nm. According to an alternative embodiment of the present invention the diamond nanoparticles have a size of at least 4 and at most 6 nm. According to a preferred embodiment of the present invention the diamond nanoparticles have a size of around 5 nm. According to a preferred embodiment of the present invention the diamond nanoparticles have a size of around 4 nm. According to a preferred embodiment of the present invention the diamond nanoparticles have a size of around 4 to 5 nm.

According to, a preferred embodiment of the present invention the diamond nanoparticles have a size of at least 0,1 nm. According to a preferred embodiment of the present invention the diamond nanoparticles have a size of at least 1 nm. According to a preferred embodiment of the present invention the diamond nanoparticles have a size of at least 2 nm. According to an alternative embodiment of the present invention the diamond nanoparticles have a size of at least 3 nm.

According to a preferred embodiment of the present invention the diamond nanoparticles have a size of at most 1000 nm. According to a preferred embodiment of the present invention the diamond nanoparticles have a size of at most 500 nm. According to a preferred embodiment of the present invention the diamond nanoparticles have a size of at most 250 nm. According to a preferred embodiment of the present invention the diamond nanoparticles have a size of at most 100 nm. According to a preferred embodiment of the present invention the diamond nanoparticles have a size of at most 50 nm. According to a preferred embodiment of the present invention the diamond nanoparticles have a size of at most 25 nm. According to an alternative embodiment of the present invention the diamond nanoparticles have a size of at most 20 nm. According to an alternative embodiment of the present invention the diamond nanoparticles have a size of at most 15 nm. According to an alternative embodiment of the present invention the diamond nanoparticles have a size of at most 10 nm. According to an alternative embodiment of the present invention the diamond nanoparticles have a size of at most 8 nm.

According to a preferred embodiment of the present invention the carbon-based nanoparticles have a terminated surface. In context with the present invention a terminated surface is a surface of a carbon-based nanoparticle, wherein the atoms at the surface are saturated by specific molecular groups or atoms. Preferably the specific molecular groups or atoms are selected from the group consisting of hydrogen, oxygen, NH₂, imides, F, S, P, biotin and mixtures thereof. Of course a skilled person knows further suitable specific molecular groups or atoms. Due to the termination of the surface the surface-energy, the wettability, the Zeta potential and/or the polarity can be altered.

According to a preferred embodiment the carbon atoms of the surface of the carbon-based nanoparticles are saturated by one specific molecular group or atoms. Alternatively, the atoms at the surface of the carbon-based nanoparticles can also be saturated with two or more different specific molecular groups or atoms.

According to a preferred embodiment of the present invention the carbon-based nanoparticles have a terminated surface leading to hydrophilic carbon-based nanoparticles. According to an alternative embodiment of the present invention the carbon-based nanoparticles have a terminated surface leading to hydrophobic carbon-based nanoparticles.

According to a preferred embodiment of the present invention the carbon-based nanoparticles are hydrophilic carbon-based nanoparticles. According to an alternative embodiment of the present invention the carbon-based nanoparticles are hydrophobic carbon-based nanoparticles.

According to a preferred embodiment of the present invention the termination of the carbon-based nanoparticle surface is oxygen-terminated, hydrogen-terminated or NH₂-terminated. According to a preferred embodiment of the present invention the termination of the carbon-based nanoparticle surface is oxygen-terminated or NH₂-terminated. According to a preferred embodiment of the present invention the termination of the carbon-based nanoparticle surface is oxygen-terminated. According to an alternative embodiment of the present invention the termination of the carbon-based nanoparticle surface is hydrogen-terminated. According to an alternative embodiment of the present invention the termination of the carbon-based nanoparticle surface is sulfur-terminated. According to an alternative embodiment of the present invention the termination of the carbon-based nanoparticle surface is nitrogen-terminated.

According to a preferred embodiment of the present invention at least one of the carbon-based nanoparticles, preferably diamond nanoparticles, has an oxygen-terminated surface. An oxygen-terminated surface is very suitable to bind biomolecules, most preferably proteins, via physisorption.

According to a preferred embodiment of the present invention at least one of the carbon-based nanoparticles, preferably diamond nanoparticles, are functionalized with a biomolecule, for example proteins or polypeptides, bound to the at least one carbon-based nanoparticle, preferably diamond nanoparticle.

According to a preferred embodiment of the present invention the biomolecule is bound to the terminated surface of the at least one carbon-based nanoparticle, preferably diamond nanoparticle.

According to a preferred embodiment of the present invention the carbon-based nanoparticles, preferably diamond nanoparticles, are functionalized with a biomolecule bound to the at least one carbon-based nanoparticle, preferably diamond nanoparticle.

In the context of the present invention "a biomolecule" means a kind of biomolecule, for example protein or polypeptide. "A biomolecule" means that at least one molecule of the biomolecule is present. Of course, and according to a preferred embodiment of the invention more than one molecule of the biomolecule can be bound to the at least one carbon-based nanoparticle, preferably diamond nanoparticle.

In the context of the present invention "bound" means covalently bound but also bound by interaction, for example by physisorption.

Of course, the at least one carbon-based nanoparticle, preferably diamond nanoparticle, can be functionalized with more than one type of biomolecule. For example the at least one carbon-based nanoparticle, preferably diamond nanoparticle, can be functionalized with two ore three or more different types of biomolecules.

In a preferred embodiment the functionalization is made by binding the biomolecules to the saturated surface, especially terminated surface of the carbon-based nanoparticles, preferably diamond nanoparticles, most preferably by physisorption.

Of course, the biocompatible porous scaffold according to the present invention can also comprise two or more different subpopulations of carbon-based nanoparticles, preferably diamond nanoparticles, wherein each subpopulation is functionalized with different types of biomolecules.

According to a preferred embodiment of the present invention the carbon-based nanoparticles, preferably diamond nanoparticles, are functionalized with a biomolecule bound to the at least one carbon-based nanoparticle, preferably diamond nanoparticle.

According to a preferred embodiment of the present invention the biomolecule is bound to the at least one carbon-based nanoparticle, preferably diamond nanoparticle, directly, for example via physisorption, or via at least one linker or functional group.

According to a preferred embodiment of the present invention the biomolecule is bound to the at least one carbon-based nanoparticle, preferably diamond nanoparticle, via physisorption, via chemisorption or via a covalent binding. Physisorption, chemisorption and covalent binding are methods known to a person skilled in the art.

According to a preferred embodiment of the present invention the biomolecule is bound to the at least one carbon-based nanoparticle, preferably diamond nanoparticle, via physisorption.

Physisorption is a non-covalent way to immobilize proteins on surfaces accomplished by physical adsorption which is based on intermolecular interactions. These intermolecular interactions are mainly hydrogen bonds, electrostatic interactions, hydrophobic and polar interactions.

General drawbacks of the physisorption is that these immobilization techniques occur random and lead to a loss of specificity, selectivity and affinity, because some molecules become inactivated during the adsorption or a fraction of the active sites is not reachable for the target molecules. Another important drawback of using physisorption is in general that these interactions are relatively weak and that the attached molecules may be removed by body fluids or by the buffers or detergents used.

The inventors surprisingly found that these drawbacks can be overcome by using terminated surfaces of carbon-based nanoparticles, especially diamond nanoparticles. The inventors found that when using such nanoparticles the physisorption between the surface of the nanoparticles and the biomolecules is strong, especially strong enough to be used in vivo and that the bioactivity of the biomolecules is preserved. Binding using physisorption has the advantage that no further chemistry like linkers, spacers, activators and so on has to be used, but that the biomolecule is bound directly to the surface of the nanoparticles.

However, in an alternative embodiment the biomolecules can be bound to the surface of the carbon-based nanoparticles, preferably diamond nanoparticles by covalent binding. This is a robust way to create biofunctional surfaces. Most methods that involve covalent coupling exploit the reactivity of endogenous functionalities of the protein. One strategy towards covalent immobilization uses these chemical groups to react with complementary chemical groups present on the substrate of interest. However, because proteins often possess multiple copies of an amino acid, again multiple protein orientations are possible. Another strategy is to modify or derivatise the natural occurring chemical groups by introducing less abandoned chemical groups or chemical linkers. In a preferred embodiment the so-called "click" chemistry coupling can be used. An example for such "click" chemistry is the copper-catalyzed azide-alkine cycloaddition. Therefore, in an alternative embodiment the biomolecules are bound to the surface of the carbon-based nanoparticles, preferably diamond nanoparticles via copper-catalyzed azide-alkine cycloaddition (CuAAC). In a further alternative embodiment the biomolecules are bound the surface of the carbon-based nanoparticles, preferably diamond nanoparticles via the short linker molecule aminocaproic acid (ACA). For the grafting of biomolecules via this linker no additional cross linker is needed. There exist many possibilities to bind proteins, via linkers at room temperature and without any additional agent. All these immobilization methods can be applied in order to achieve the binding of the biomolecule in the scaffold via carbon-based particles.

According to a preferred embodiment of the present invention the biomolecule is a polypeptide or a protein. According to a preferred embodiment of the present invention the biomolecule is a growth factor. According to an alternative embodiment of the present invention the biomolecule can be a component of the extracellular matrix.

According to a preferred embodiment of the present invention the biomolecule is a growth factor. According to an alternative embodiment of the present invention the biomolecule is an osteogenesis related factor.

According to a preferred embodiment of the present invention the biomolecule is selected from the group consisting of VEGF, pIGF, G-CSF, PDGF-BB, KGF, heparin, Ang-1 and BMP-2. Of curse, a person skilled in the art knows further suitable biomolecules, especially osteogenesis related factors.

According to a preferred embodiment of the present invention the biomolecule is Ang-1 or BMP-2 or VEGF.

According to a preferred embodiment of the present invention the biomolecule is Ang-1. According to a preferred embodiment of the present invention the biomolecule is BMP-2. According to a preferred embodiment of the invention the biomolecule is VEGF.

In an alternative embodiment of the present invention of course also mixtures of different biomolecules can be bound to the surface of the carbon-based nanoparticles.

In an alternative embodiment of the present invention a biomolecule mixture of Ang-1 and BMP-2 or a biomolecule mixture of Ang-1 and VEGF or a biomolecule mixture of BMP-2 and VEGF or a biomolecule mixture of Ang-1 and BMP-2 and VEGF is used.

According to an alternative embodiment of the present invention the biomolecule is a medicament. According to an alternative embodiment of the present invention the biomolecule is an antibody. According to an alternative embodiment of the present invention the biomolecule is a hormone. According to an alternative embodiment of the present invention the biomolecule is an antibiotic. According to an alternative embodiment of the present invention the biomolecule is a vitamin.

Of course also a mixture of biomolecules like osteogenesis-related factors can be combined and mixed with medicaments, antibodies or vitamins.

The present invention also relates to the biocompatible porous scaffolds according to the present invention comprising carbon-based nanoparticles, preferably diamond nanoparticles, which are not functionalized with biomolecules. According to a preferred embodiment of the present invention nanoparticles which are not functionalized with biomolecules have a terminated surface. Due to the presence of carbon-based nanoparticles, preferably diamond nanoparticles, which are not functionalized with biomolecules but have a terminated surface the scaffolds can easily get functionalized with biomolecules, for example by giving the biomolecules to the scaffold and binding the biomolecules to the nanoparticles, preferably by physisorption.

In the context of the present invention the biocompatible porous scaffold is a three-dimensional scaffold.

In the context of the present invention the biocompatible porous scaffold is made from at least one scaffold material which is biocompatible and porous. Preferably the scaffold material is a graft material, preferably a natural or synthetic graft material. Preferably the scaffold material is an alloplastic, xenogenic or allogenic material.

According to a preferred embodiment of the present invention the biocompatible porous scaffold is a tissue regeneration scaffold.

According to a preferred embodiment of the present invention the biocompatible porous scaffold is a bone regeneration scaffold.

Bone regeneration scaffolds are also known as artificial bone. Bone regeneration scaffold materials are also known bone-like materials or bone graft substitute materials.

According to a preferred embodiment of the present invention the at least one biocompatible porous scaffold material is an inorganic material. According to an alternative embodiment of the present invention the at least one biocompatible porous scaffold material is an organic material. According to a preferred embodiment of the present invention the at least one biocompatible porous scaffold material is biologically inert.

According to a preferred embodiment of the present invention the at least one biocompatible porous scaffold material is biodegradable. In an alternative embodiment the at least one biocompatible porous scaffold material is not biodegradable.

The person skilled in the art knows a great number of materials which are suitable to be used as biocompatible porous scaffold material or which are used in the art as biocompatible porous scaffold material.

According to a preferred embodiment of the present invention the at least one biocompatible porous scaffold material is selected from the group consisting of a ceramic material, a mineral, bone, metal, metal foam, a magnesium compound an organic compound, preferably collagen or bone, more preferably autologous bone, a composite material, a silicium based semiconductor, a silica, a silica glass, a carbon-based material or mixtures thereof.

According to an alternative embodiment of the present invention the at least one biocompatible porous scaffold material is a polymer. According to an alternative embodiment of the present invention the at least one biocompatible porous scaffold material is a ceramic material. According to an alternative embodiment of the present invention the at least one biocompatible porous scaffold material is a mineral. According to an alternative embodiment of the present invention the at least one biocompatible porous scaffold material is bone. According to an alternative embodiment of the present invention the at least one biocompatible porous scaffold material is a metal. According to an alternative embodiment of the present invention the at least one biocompatible porous scaffold material is a metal foam. According to an alternative embodiment of the present invention the at least one biocompatible porous scaffold material is a magnesium compound. According to an alternative embodiment of the present invention the at least one biocompatible porous scaffold material is a organic compound. According to an alternative embodiment of the present invention the at least one biocompatible porous scaffold material is collagen. According to an alternative embodiment of the present invention the at least one biocompatible porous scaffold material is bone, preferably autologous bone. According to an alternative embodiment of the present invention the at least one biocompatible porous scaffold material is a composite material. According to an alternative embodiment of the present invention the at least one biocompatible porous scaffold material is a silicium based semiconductor. According to an alternative embodiment of the present invention the at least one biocompatible porous scaffold material is a silica or a silica glass. According to an alternative embodiment of the present invention the at least one biocompatible porous scaffold material is a carbon-based material.

According to a preferred embodiment of the present invention the at least one biocompatible porous scaffold material contains calcium.

According to a preferred embodiment of the present invention the at least one biocompatible porous scaffold material is a calciumphosphate.

According to a preferred embodiment of the present invention the at least one biocompatible porous scaffold material is β-tricalciumphosphate.

According to a preferred embodiment of the present invention the at least one biocompatible porous scaffold material is CeraSorb^{®}. According to a preferred embodiment of the present invention the at least one biocompatible porous scaffold material is ChronOS^{®}.

In an alternative embodiment of the present invention the at least one biocompatible porous scaffold material is polypropylene. A person skilled in the art knows further suitable materials and material-combinations, for example polymers, homopolymers, copolymers, alloys. In an alternative embodiment of the present invention the at least one biocompatible porous scaffold material is based e.g. on caprolactone or lactide etc.

Of course, the different biocompatible porous scaffold materials can be used alone or in mixture. The biocompatible porous scaffold can comprise one, two, three or more of biocompatible porous scaffold materials, preferably of the biocompatible porous scaffold materials outlined in the present description. For example the material of the biocompatible porous scaffold can be a mixture of hydroxylapatite and tricalciumphosphate, for example 60 % hydroxylapatite and 40 % tricalciumphosphate.

According to a preferred embodiment of the present invention the at least one biocompatible porous scaffold material is a synthetic scaffold material, preferably synthetic beta-tricalciumphosphate or synthetic hydroxylapatite.

According to a preferred embodiment of the present invention the at least one biocompatible porous scaffold is nano-porous. According to a preferred embodiment of the present invention the at least one biocompatible porous scaffold is micro-porous. According to a preferred embodiment of the present invention the at least one biocompatible porous scaffold is meso-porous. According to a preferred embodiment of the present invention the at least one biocompatible porous scaffold is macro-porous.

A person skilled in the art knows typical and suitable pore sizes of scaffolds used for tissue regeneration, especially bone regeneration. Preferably most of the pores of the biocompatible porous scaffold have a pore size of at least 20 µm and at most 600 µm, for example of at least 50 µm and at most 500 µm. In an alternative embodiment the pore size can be also of at least 500 µm to at most 2000 µm.

Preferably, the pore size is at least 20 µm, more preferably at least 50 µm. In a preferred embodiment of the present invention the pore size is at most 2500 µm, preferably 2000 µm, in an alternative embodiment at most 1000 µm, preferably 500 µm.

The scaffold can have also a pore size of 50 µm to 1000 µm, for example 50 µm to 150 µm or 150 µm to 500 µm or 500 µm to 1000 µm or 1000 µm to 2000 µm.

In the context of the present invention "pore size" means the pore size of most of the pores of a porous scaffold. Of course, some of the pores of the porous scaffold can have a smaller or a bigger pore size,

In a preferred embodiment of the present invention the biocompatible porous scaffold has a porosity of at least 50 %, more preferably of at least 60 %, for example of 60 to 90 %.

The biocompatible porous scaffold can have various sizes and forms. The scaffold can have for example a size of 0.2 mm to 2 mm. A scaffold with such a size is preferably provided as part of a granulate comprising a plurality of scaffolds. The scaffold can alternatively have also the form of a block or of a membrane having a length for example up to 10 cm or more, preferably up to 2 cm, preferably 1 cm, more preferably 50 mm.

The biocompatible porous scaffold can have for example the form of a granulate, a block, a membrane, a cylinder, a wedge or a ring or combinations of them, for example a combination of a first scsaffold material having the form of a cylindric ring filed with a second scaffold material in the form of a cylinder or being in form of a granulate.

According to a preferred embodiment of the present invention the carbon-based nanoparticles are applied to the biocompatible porous scaffold material by perfusion, injection, dip-coating, spin-coating, electro-spraying or vaporization in vacuum or other techniques for implementation of particles in porous materials. A person skilled in the art knows such suitable methods which ensure that the pores are not blocked by the carbon-based nanoparticles. This can be monitored for example by the use of fluorescence-labeled carbon-based nanoparticles.

According to the present invention the biocompatible porous scaffold can comprise one or more different kinds of carbon-based nanoparticles.

According to a preferred embodiment of the present invention the carbon-based nanoparticles are bound to at least a part of the surface of the biocompatible porous scaffold material.

The carbon based nanoparticles can be bound mechanical for example by partial incorporation of the nanoparticles into the scaffold or by fixation via chemical surface-groups or via linkers. The carbon based nanoparticles can be bound covalently or by adsorption.

According to a preferred embodiment of the present invention the carbon-based nanoparticles are present as particles on the surface of the biocompatible porous scaffold material.

According to a preferred embodiment of the present invention the carbon-based nanoparticles are not present as a layer or film covering at least a part of the surface of the porous scaffold material.

According to a preferred embodiment of the present invention the carbon-based nanoparticle-layer is an electrically isolating layer. In an alternative embodiment the carbon-based nanoparticle-layer is an electrically conductive layer.

According to an alternative embodiment of the present invention the carbon-based nanoparticles are embedded into the biocompatible porous scaffold material. The carbon-based nanoparticles can be embedded for example in the scaffold material base substance from which the biocompatible porous scaffold is formed or otherwise produced, for example by polymerization or drying. This means that the biocompatible porous scaffold according to the present invention can be produced, especially formed, from a mixture of the biocompatible porous scaffold material and the carbon-based nanoparticles. This concept improves the hydrophilicity or mechanical stability of the original material. The biocompatible porous scaffold can be produced for example by electrospinning or 3-dimensional fiber deposition method (e.g. Fedorovich NE et al: "Three-dimensional fiber deposition of cell-laden, viable, patterned constructs for bone tissue printing"; Tissue Eng Part A. (2008) ;14(1):page 127 to page 33).

In a further alternative embodiment the biocompatible porous scaffold material is solved, preferably colloidal solved, and afterwards dried together with the carbon-based nanoparticles. The solvent can be for example water, buffer, cell culture media, body-fluids, more preferably blood, but can be for example also a gas.

According to the present invention the carbon-based nanoparticles, preferably diamond nanoparticles, are present as particles in the biocompatible porous scaffold, especially on the surface of the biocompatible porous scaffold. This means that the nanoparticles do not form a film or layer on the surface of the scaffold, but are present as single spots. Therefore, there are gaps in between the different carbon-based nanoparticles, preferably diamond nanoparticles, so that substances, and especially fluids, can reach not only the carbon-based nanoparticles, preferably diamond nanoparticles, but also the surface of the biocompatible porous scaffold. This is important, especially in the case wherein a biodegradable scaffold material is used. If the nanoparticles would form a film, a biodegradation of the scaffold would not be possible or would be at least delayed, since body fluids like blood cannot reach the scaffold due to the nanoparticle cover. The presence of the nanoparticles in form of distinct particles is in contrast to the nanoparticle films and coatings of implants, for example implants consisting of a metal like titanium, where the nanocrystalline diamond film or layer is used also to avoid the contact between the tissue and/or body fluid and the metal surface of the implant.

According to a preferred embodiment of the present invention the distance between carbon-based nanoparticles, preferably diamond nanoparticles, on the surface of the biocompatible porous scaffold is wide enough to allow the biodegradation of the scaffold, i. e. to allow the infiltration of the enzymes responsible for the biodegradation in the clearances between the nanoparticles so that these enzymes can reach the scaffold surface. In a preferred embodiment of the invention the distances between the nanoparticles can be altered in different areas of the scaffold, resulting in different biodegradation-times of the different areas. Accordingly a part of the scaffold can be covered by the nanoparticles having nearly no distances in-between the nanoparticles, resulting in a very late biodegradation of this part of the scaffold.

In a preferred embodiment of the present invention the carbon-based nanoparticles, preferably diamond nanoparticles, on the surface of the biocompatible porous scaffold, preferably biodegradable, biocompatible porous scaffold have a clearance of at least 10 nm, more preferably of at least 50 nm.

In general, the presence and distribution of the carbon-based nanoparticles can be analyzed for example by the use of fluorescence-labelled carbon-based nanoparticles. Either the carbon-based nanoparticles used are fluorescence-labeled or a second type of carbon-based nanoparticles, which are fluorescence-labeled, are used additionally.

The carbon-based nanoparticles can be distributed homogenously in the biocompatible porous scaffold material. In an alternative embodiment the carbon-based nanoparticles are distributed heterogeneously, especially as a gradient in the biocompatible porous scaffold material. In a further embodiment of the present invention different carbon-based nanoparticles, preferably carbon-based nanoparticles with different functionalization can be distributed in the biocompatible porous scaffold material. In a further embodiment of the present invention carbon-based nanoparticles with different functionalization can be distributed in different areas of the biocompatible porous scaffold.

The concentration of the carbon-based nanoparticles being present in the biocompatible porous scaffold depends from the demand of the intended use, for example from the mechanical stability or the porosity of the surface of the biocompatible porous scaffold.

In a further alternative embodiment the carbon-based nanoparticles are present in the pores of the biocompatible porous scaffold. In a further alternative embodiment the carbon-based nanoparticles are present only in the pores of the biocompatible porous scaffold.

According to a preferred embodiment of the present invention the biocompatible porous scaffold comprises the carbon-based nanoparticles in an amount of at least 0,01 % by weight to at most 10 % by weight. According to a preferred embodiment of the present invention the biocompatible porous scaffold comprises the carbon-based nanoparticles in an amount of at least 0,1 % by weight to at most 10 % by weight. According to a preferred embodiment of the present invention the biocompatible porous scaffold comprises the carbon-based nanoparticles in an amount of at least 0,5 % by weight to at most 5% by weight.

According to a preferred embodiment of the present invention the biocompatible porous scaffold comprises the carbon-based nanoparticles in an amount of at least 1 % by weight to at most 3 % by weight, more preferably of around 2% by weight.

According to a preferred embodiment of the present invention the biocompatible porous scaffold comprises the carbon-based nanoparticles in an amount of at least 0,01 % by weight. According to a preferred embodiment of the present invention the biocompatible porous scaffold comprises the carbon-based nanoparticles in an amount of at least 0,1 % by weight. According to a preferred embodiment of the present invention the biocompatible porous scaffold comprises the carbon-based nanoparticles in an amount of at most 10 % by weight.

According to a preferred embodiment of the present invention the biocompatible porous scaffold is a tissue regeneration scaffold.

According to a preferred embodiment of the present invention the biocompatible porous scaffold is a bone regeneration scaffold.

The present invention also refers to a biocompatible porous scaffold according to the present invention for the use of treating a tissue defect, preferably a bone defect.

The present invention solves its underlying technical problem in particular also by a granulate comprising at least two biocompatible porous scaffolds according to the present invention, preferably a plurality of biocompatible porous scaffolds according to the present invention.

The present invention solves its underlying technical problem in particular also by the use of carbon-based nanoparticles for the production of a biocompatible porous scaffold, especially a biocompatible porous scaffold according to the present invention.

According to a preferred embodiment of the present invention the used carbon-based nanoparticles are diamond nanoparticles.

The present invention solves its underlying technical problem in particular also by a method for the production of a biocompatible porous scaffold according to the present invention, wherein a biocompatible porous scaffold material is combined with carbon-based nanoparticles.

In a preferred embodiment of the present invention the method comprises the steps a) modifying a biocompatible porous scaffold with a solution of carbon-based nanoparticles, more preferably diamond nanoparticles, wherein preferably the solvent is deionized water, and b) sterilizing the modified biocompatible porous scaffold. The modification can be made for example via injection, perfusion, dip coating, spraying and so on. The sterilization can be made for example by gamma sterilization, treating by autoclave, treating with UV light, gas sterilization or using an electron beam.

In a preferred embodiment of the methods according to the present invention the carbon-based nanoparticles or diamond nanoparticles used have a terminated surface, but are not functionalized with biomolecules. Alternatively, the biocompatible porous scaffold can be modified also directly with carbon-based nanoparticles, preferably diamond nanoparticles, which are already functionalized with biomolecules.

The present invention solves its underlying technical problem in particular also by a method for treating a tissue defect, wherein at least one tissue regeneration scaffold according to the present invention is filled into the tissue defect.

The present invention solves its underlying technical problem in particular also by a method for treating a bone defect, wherein at least one bone regeneration scaffold according to the present invention is filled into the bone defect.

The present invention also refers to a method for the production of a biocompatible porous scaffold having at least one carbon-based nanoparticle, preferably diamond nanoparticle, being functionalized with a biomolecule bound to the at least one carbon-based nanoparticle, preferably diamond nanoparticle, comprising the step of binding the biomolecule to the at least one carbon-based nanoparticle of the biocompatible porous scaffold. Preferably the binding is done via physisorption.

The present invention also refers to a kit of parts comprising at least one biocompatible porous scaffold, at least one type of carbon-based nanoparticles, preferably of functionalized carbon-based nanoparticles and an instruction manual.

The present invention also refers to a kit of parts comprising at least one biocompatible porous scaffold comprising a biocompatible porous scaffolds material and carbon-based nanoparticles, wherein the carbon-based nanoparticles, preferably diamond nanoparticles, are not functionalized with a biomolecule. In a preferred embodiment of the present invention the biocompatible porous scaffold of the kit of parts is a biocompatible porous scaffold according to the present invention. Accordingly, the carbon-based nanoparticles are preferably diamond nanoparticles, preferably wit a terminated surface.

In a preferred embodiment the kit of parts contains at least two, more preferably a plurality of, biocompatible porous scaffolds comprising a biocompatible porous scaffold material and carbon-based nanoparticles, wherein the carbon-based nanoparticles, preferably diamond nanoparticles, are not functionalized with a biomolecule.

In a preferred embodiment the kit of parts contains an instruction manual in which it is explained how biomolecules can be bound to the carbon-based nanoparticles, more preferably can be bound to the carbon-based nanoparticles via physisorption.

A kit of parts comprising at least one biocompatible porous scaffold comprising a biocompatible porous scaffold material and carbon-based nanoparticles, wherein the carbon-based nanoparticles, preferably diamond nanoparticles, are not functionalized with a biomolecule, i.e. a kit of parts comprising no biomolecules but comprising a scaffold comprising carbon-based nanoparticles has the advantage that it can be stored easily and for a long time but can be functionalized with biomolecules direct before use easily for example due to physisorption.

The present invention also refers to a kit of parts comprising a) at least one biocompatible porous scaffold comprising a biocompatible porous scaffold material and carbon-based nanoparticles, wherein the carbon-based nanoparticles, preferably diamond nanoparticles, are not functionalized with a biomolecule and b) at least one biomolecule, wherein the at least one biocompatible porous scaffold and the at least one biomolecule are physically separated in the kit of parts. Preferably the kit of parts comprises an instruction manual. Preferably the at least one biocompatible porous scaffold and the at least one biomolecule are present in the kit of parts in different packages, for example boxes, containers and/or bags. In a preferred embodiment of the present invention the biocompatible porous scaffold of the kit of parts is a biocompatible porous scaffold according to the present invention. Accordingly, the carbon-based nanoparticles are preferably diamond nanoparticles.

According to a preferred embodiment of the present invention the carbon-based nanoparticles, preferably the diamond nanoparticles, have a terminated surface. More preferably the carbon-based nanoparticles, preferably diamond nanoparticles, have a hydrophilic surface. Even more preferably the carbon-based nanoparticles, preferably diamond nanoparticles, have an oxygen-terminated surface.

In a preferred embodiment of the present invention the at least one biocompatible porous scaffold provided in the kit of parts is sterilized. A biocompatible porous scaffold according to the present invention which is not functionalized, but has carbon-based nanoparticles, preferably diamond nanoparticles with a terminated surface, can be used, especially when provided in a kit of parts as presently disclosed in an easy and safe way by a physician. The scaffold is provided non-functionalized so that the possibility to store the scaffold is improved, since no biomolecules are present. When using the scaffold the physician can then functionalize the scaffold as demanded. Especially the physician can functionalize the scaffold without the help of technicians using the provided kit of parts. The functionalization of the scaffold is especially then very easy for a physician when the biomolecules are bound to the surface of the carbon-based nanoparticles, preferably diamond nanoparticles, via physisorption. This can be done directly prior a surgery. Furthermore, the physician can decide on his own and from case to case with which biomolecules the scaffold shall be functionalized and which amounts of biomolecules shall be used. Preferably, the kit of parts comprises an instruction manual showing how to functionalize the biocompatible porous scaffold via the carbon-based nanoparticles, preferably diamond nanoparticles, and outlining suitable concentrations of the biomolecules.

With the preferred kit of parts the present invention provides a tool for tissue regeneration, especially bone regeneration, which can be stored without any problems and for a long time, since the scaffold and the biomolecules are stored in different containers and with which the scaffold can be functionalized easily and individually from case to case. Furthermore the biocompatible porous scaffold comprising carbon-based nanoparticles, preferably diamond nanoparticles, preferably with a terminated surface, but no biomolecules is not necessarily a medicament.

In the context of the present invention all relative amounts given in percentage (%) of an indicated overall composition add up to 100 % of the indicated overall composition.

In the context of the present invention the wordings "to comprise" and "to contain" and their conjugations are used in one preferred embodiment in their non-limiting sense to mean that items following the wording are included, but items not specifically mentioned are not excluded. In the context of the present invention the wording "to comprise" or "to contain" and their conjugations are used in another preferred embodiment in its limiting sense to mean that items following the wordings are included and items not specifically mentioned are excluded thereby equaling the meaning of the wording "to consist" and its conjugations.

Reference to an element of the present invention, particularly biocompatible porous scaffold, carbon-based nanoparticle, diamond based nanoparticle or method, by the indefinite article "a" or "an" does not exclude the possibility that more than one of the element is present, unless the context clearly requires that there be one and only one of the elements. The indefinite article "a" or "an" thus usually means "at least one".

Reference to "at least one" element of the present invention, particularly biocompatible porous scaffold, carbon-based nanoparticle or diamond based nanoparticle does include the presence of one of the element or more of the elements, for example two, three or more of the elements, preferably a plurality of the element. In the context of the present invention "at least one" element, for example nanoparticle, can also mean all elements, for example nanoparticles.

Further preferred embodiments are the subject matter of the subclaims.

The invention will be further described by way of non-limiting examples and the figures.
Figure 1 shows a typical size distribution of diamond nanoparticles.
Figure 2 shows a typical size distribution of diamond nanoparticles used in the examples before and after the physisorption of the biomolecule Ang-1 (figure 2A) and the Zeta potential of the diamond nanoparticles before (figure 2B) and after (figure 2C) the physisorption of the biomolecule Ang-1.
Figure 3 shows the protein release comparison from pure CeraSorb^{®} scaffolds and hydrophilic nano-diamond particle modified CeraSorb^{®} scaffolds.
Figure 4 shows the quantification of blood vessel density within the overall area of ChronOS^{®} scaffolds and the central region of ChronOS^{®} scaffolds after 4 weeks post-implantation of (A) scaffolds without nano-diamond particles (nDP), (B) of scaffolds with unfunctionalized nano-diamond particles and (C) of scaffolds with functionalized nano-diamond particles. *P<0.05 and **P<0.01 indicates significant differences relative to the scaffolds without nano-diamond particles (n=5 for each the group). Data is presented as mean±s.d..
Figure 5 shows two examples of a cylindrical two compound scaffold.

### Example 1: Preparation terminated diamond nanoparticles

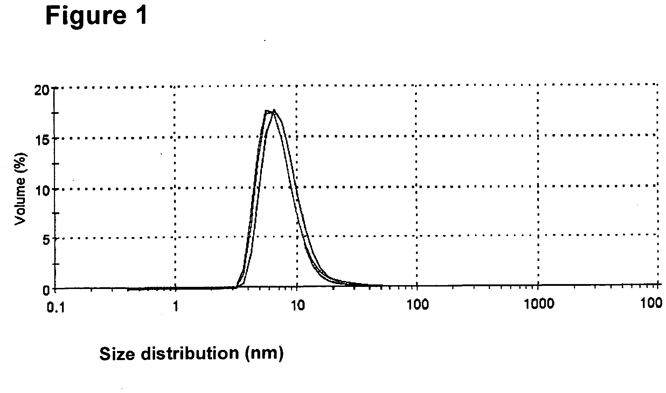

Preparation of the colloidal nano-crystalline-diamond particles (nDP), purchased from Gansu Lingyun Corp. (China)) was performed using a special "milling" technique for initial ultradispersed diamond particles in order to achieve a narrow size distribution and low agglomeration of the particles. After milling purification with saturated sodium hydroxide solution and hydrochloric acid (conc.), redispersing was performed in ultrasonic equipment which resulted in a colloidal particle solution with lowest agglomeration (see figure 1). Afterwards nDPs were treated with oxygen in order to exhibit chemical groups on the surface which influence the surface energy and thus the hydrophilicity of the diamond particles. In the applied nDP the hydrophilic surface was achieved by increasing OH and COOH-groups due to the following process:
acid etching with sulphuric acid/nitric acid/perchloric acid 1:1:1 (each conc.) at 80°C and washing with water for neutralization.
Fourier-transform infrared spectrometry (FTIR) was carried out as described in Ghodbane S. et al.: "HREELS investigation of the surfaces of nanocrystalline diamond films oxidized by different processes", Langmuir, (2010); 26 (24), page 18798 to page18805. FTIR spectrum recorded from the surface of these nDP shows the presence of several carbon-oxygen groups (C-O, C=O, O-C-O) as well as a very low concentration of carbon-hydrogen (CHx) groups (data not shown).

To get an estimation of the hydrophilicity of the terminated nano-diamond particles a dip coating of the nDP solution (liquid: deionized water) was prepared on Teflon. Contact angle measurements of the pure Teflon surface (120°) and the dip coating (8°) confirmed the hydrophilic behavior of the nDP surface (data not shown).

### Example 2: Modification of biocompatible porous scaffolds with terminated diamond nanoparticles

The modification of ChronOS^{®} or CeraSorbM^{®} scaffolds in form of cylinders with the well characterized n-DP solution was carried out using perfusion technique and drying in vacuum. Due to the fact that liquid in vacuum immediately bursts into gaseous form and then begins to form an equilibrium between its vapour and liquid form the process parameters have been developed and optimized. Depending on vacuum pressure, velocity of pumping down and vapour pressure the "explosion" of the n-DP-liquid surface tension and thus the distribution into the scaffold can be controlled.

The particles were well distributed even within the center of the scaffolds.

The concentration of the nDP-solution was (2 wt. % and the particle size distribution was measured with Malvern-Zetasizer Nano-ZS resulting in a narrow distribution with its maximum at 5 - 7 nm, i.e. 0,125 cts/ml, i.e. 10¹⁷ nano-diamond particles/ml).The concentration can be adjusted to the needs of the application.

The scaffolds were gamma-sterilized after the modification.

In order to visualize the distribution of nano-diamond particles (nDP) within the ChronOS^{®} (Synthes, Austria), a βtricalcium phosphate (β-TCP) scaffold, Oregon-green fluorescent dye was grafted to the nDP surface via amide bond formation onto arylated nanodiamond. Oregon-green labeled nDP was perfused into the ChronOS scaffold with procedures described in section 2.2. After embedding of the specimen in Technovit 9100 New^{®}, a section with 40µm thickness was obtained via sawing, grinding and polishing techniques. The distribution of fluorescent labelled nDP was then observed by a fluorescent microscope (Eclipse 80i, Nikon) at 492-510nm. The resulting pictures (data not shown) show a particulate distribution of the nDP on the surface of the scaffold and also on the surface of the pores of the scaffold.

Example 3: Functionalization of diamond nanoparticles on biocompatible porous scaffolds with Ang-1 via physisorption

Human recombinant Ang-1 (R&D System, Germany) was first reconstructed in 1x Dulbecco's phosphate buffered saline (Sigma, Germany).

Required amount of protein diluted in solution (depending on the size of the scaffold and the application) was given to scaffolds with terminated diamond nanoparticles. The scaffolds were shaked for 60 minutes at room temperature. Afterwards three washing cycles with buffer or sterilized/distilled water have been executed to solve the unbound protein.

In a further experiment, prior to physisorption of Ang-1 to the ChronOS^{®} scaffold, the protein was labelled with fluorescent Rhodamine red using a Lightening-link conjugation kit (Innova Bioscensces, UK) by following the manufacture's protocols. To visualize the presence of Ang-1 within scaffold, 1ml Rhodamine-labelled Ang-1 with 1µg/ml concentration was injected into the ChronOS^{®} and ChronOS+nDP scaffold following by 30min incubation at room temperature and rinsed with PBS. Slides with 40µm thickness were obtained with the procedures described in section 2.5. The samples were then observed by a fluorescent microscope at 570-590nm.

To determine the amount of Ang-1 within the scaffold, same procedures were performed as described above except of using fluorescence-labeled Ang-1. The washing solution (3 times of 1ml PBS solution), containing unbound proteins, was collected. The amount of Ang-1 remaining in the washing solution was detected using an immunoassay kit (ELISA Quantikine Human Angiopoietin-1 Immunoassay, R&D System) by following manufacture's instructions.

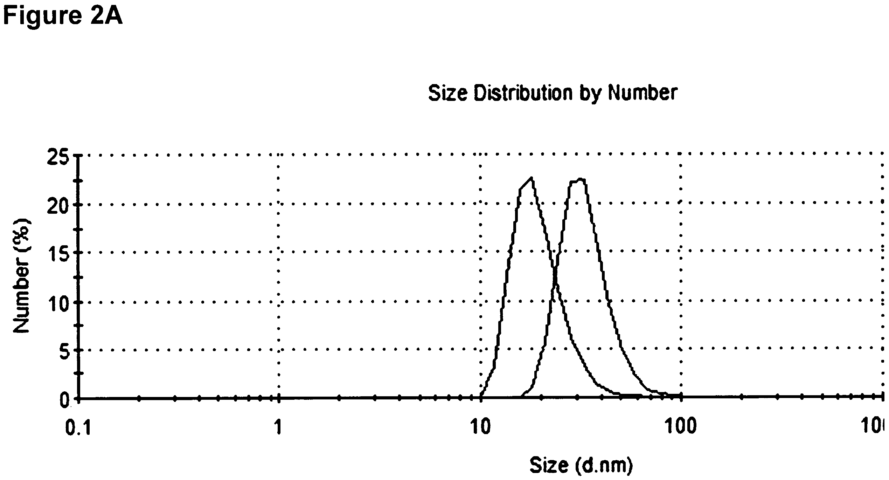

As shown in figure 2A the size distribution of the diamond nanoparticles is before the physisorption of Ang-1 around 19 nm and after the physisorption of Ang-1 around 30 nm. The increase in size value is caused by the addition of the Ang-1 molecules, which have a size of around 5 nm.

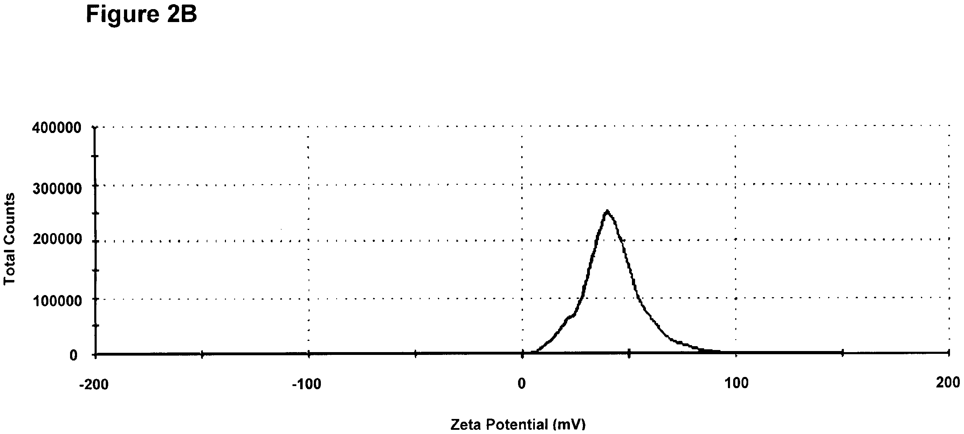

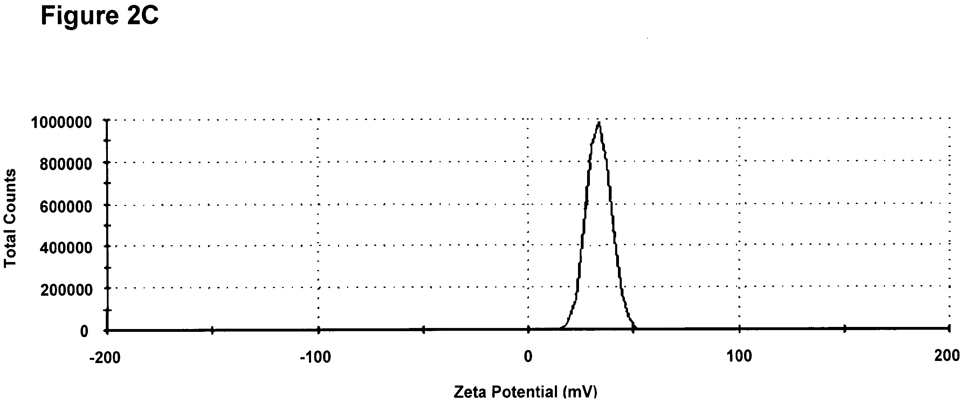

As shown in figures 2B and 2C the Zeta potential of the diamond nanoparticles decreases due to the physisorption of Ang-1 from around +40 mV (figure 2B) to around + 34 mV (figure 2C). However a Zeta potential between ± 30 mV and ± 60 mV leads to a good stability, i.e. repulsion between the particles in the solution is high enough to avoid any significant agglomeration.

### Example 4: Protein release comparison from pure scaffolds and hydrophilic nano-diamond particle modified scaffolds

To determine the amount of protein physisorbed in the n-DP modified scaffold a releasing test was implemented. Into a well defined test volume of CeraSorbM^{®} scaffold (1 cm³) 5 µg of a Ang-1, bovine serum albumin BSA diluted in 200 µl H₂O have been injected/physisorbed in pure and n-DP modified CeraSorbM^{®}. This amount is not titrated for the well defined volume of the scaffold. After washing, the scaffolds were kept in a water solution on a shaker for 7 days.

The protein content was measured from the washing solution at different time-point using an ELISA microplate reader via Coomassie blue^{®} colorimetric assay. After 7 days the amount of protein released from the pure scaffold was around 90% whereas only 20% of protein were released from the modified one.

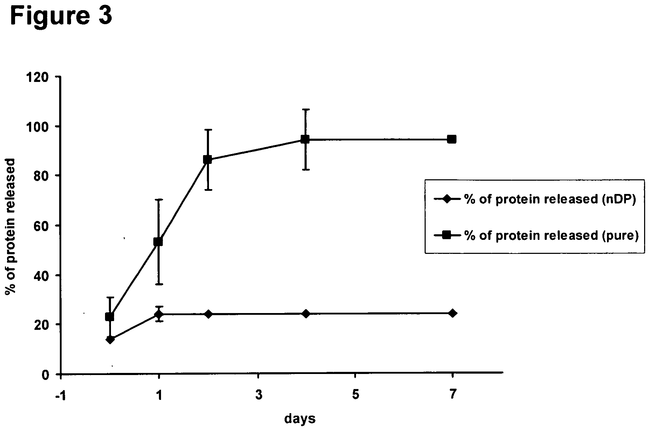

Accordingly, as shown in figure 3, the biomolecule has a strong binding to the diamond nanoparticles via physisorption, but not to the surface of the scaffold.

### Example 5: In vivo experiment with critical size bone defects demonstrating the biological activity of the functionalized scaffolds

The animal experiment was performed after approval by the national government and authorities (BMWF-66.011/0146-II/3b/2010). Healthy six-year-old female sheep weighing 75 ±5kg were fasted overnight while having free access to water. 0.5mg atropine was administered and anaesthesia was induced with ketamin (Ketavet 7-8mg/kg body weight). After fiberoptic intubation during spontaneous respiration, the sheep were ventilated with a volume controlled ventilator (Draeger EV-A, Germany) with 35% O₂/air at 14-18 breaths/min and a tidal volume of 800 ml. Anaesthesia was maintained with Isoflurane (Forane^{®}, Abbot, Austria) and Ringer's solution (6ml/kg/h) was administered continuously throughout the operation to replace fluid loss during the surgical procedure. A sagittal incision was carried out on the forehead to access the frontal bone. Critical size defects (1 cm diameter) were created with a trephine burr, care was taken to keep a distance from defect to defect of 1 cm. The defects were fully penetrating. During trepanation, special care was taken not to damage the dura. Defects were filled either with ChronOS, ChronOS+nDP, ChronOS+nDP+Ang-1 or left unfilled as control. Before suturing the periosteum was removed above the defects. The sheep were sacrificed after 4 weeks. Neovascularization was evaluated by von Willebrand immunohistochemical staining.

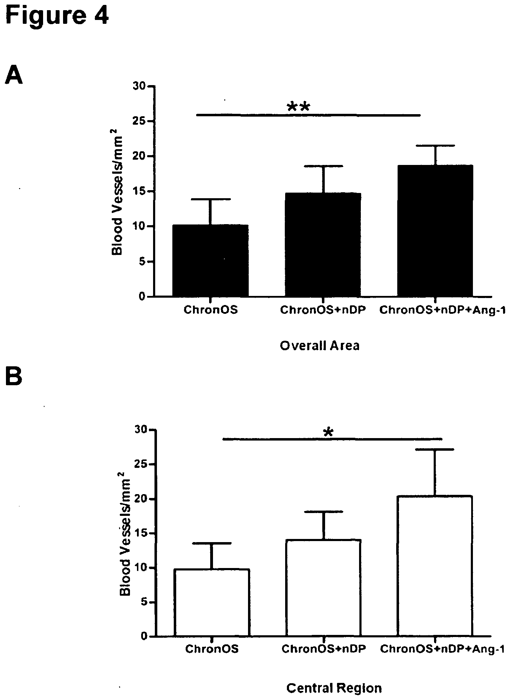

The results are shown in figure 4. The ChronOS+nDP+Ang-1 demonstrated significantly greater neovascularization in the defect (18.7±2.8 vessels/mm², p<0.01) compared to the ChronOS group (10.2±3.7 vessels/mm²). The blood vessels were not only interspersed within the pores of ChronOS+nDP+Ang-1 but also infiltrated inside the micropores of the scaffold's struts (data not shown). Interestingly, the central region of the ChronOS+nDP+Ang-1 also showed significantly higher vessel density (20.4±6.8 vessels/mm², p<0.05) compared with ChronOS scaffold (9.8±3.8 vessels/mm²). The enhanced neovascularization indicates that physisorbed Ang-1 in ChronOS+nDP scaffold was highly active to promote vessel formation. On the other hand, the vessel density of the ChronOS+nDP scaffold (in either overall area or central region was higher than the ChronOS, but lower than the ChronOS+nDP+Ang-1 scaffold.

### Example 6: Cylindrical two compound scaffold

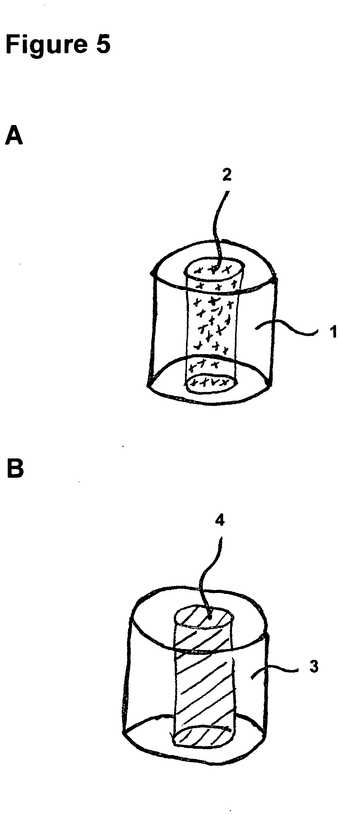

A scaffold according the present invention as shown in figure 5 has the form of a cylinder comprising two different scaffold materials, a) a hard scaffold material, for example ChronOS^{®} or CeraSorbM^{®} and b) a soft scaffold material, for example DXO. In one embodiment the hard scaffold material is building the outer part (1) of the scaffold and the soft material is building the inner (2) part of the scaffold (figure 5A). In this embodiment the soft material could also be in form of granulate. The hard material scaffold comprises diamond nanoparticles which could bind BMP-2 via physisorption. The soft material scaffold comprises diamond nanoparticles which could bind Ang.-1/VEGF via physisorption. Such a scaffold could be used for bone regeneration of long bones like the femur.

In a further embodiment the soft scaffold material is building the outer part (3) of the scaffold and the hard material is building the inner part (4) of the scaffold (figure 5B). The soft material scaffold comprises diamond nanoparticles which could bind BMP-2 via physisorption. The hard material scaffold comprises diamond nanoparticles which could bind Ang.-1/VEGF via physisorption. Such a scaffold could be used for bone regeneration of the scull or jaw.

### Example 7: Kit of parts for bone regeneration and its use

The novel, sterilized n-DP modified scaffold (with defined groups on the diamond surface, e.g. OH, COOH, NH₂, etc. depending on the application - wettability, subsequent physisorption) will be supplied to the clinicians/doctor in a kit of parts. The kit of parts can also include separately specific biomolecules, for example growth factors. Before surgery the clinician decides whether an additional growth factor or combinations of growth factors are required (depending on the defect size, age, condition of the patient etc.) and prepares the proteins, cocktails, doses etc.

One hour before surgery the required growth factor will be preferably physisorbed or alternatively covalently bond (using click chemistry or linker on the n-DP for immediate reaction at room temperature) to the sterile n-DP-scaffold. The following procedure can be used: The n-DP scaffolds are shaked in the required amount of protein in solution (buffer, PBS, deionized water, etc.) for 30 - 60 minutes at room temperature. Afterwards three washing cycles with puffer or sterilized/distilled water have been executed to solve the unbound protein.

Now the active implant can be applied to the patient.

Alternatively the clinician can only use the n-DP-modified scaffold without additional growth factors. Just the increase of hydrophilicity improves the bone ingrowth or tissue development.

The scaffold can be a single material or a combination of different porous biomaterials, granulates etc.

## Claims

1. Biocompatible porous scaffold comprising a biocompatible porous scaffold material and carbon-based nanoparticles.

2. Biocompatible porous scaffold according to claim 1, wherein the carbon-based nanoparticles are diamond nanoparticles.

3. Biocompatible porous scaffold according to the preceding claims, wherein the biocompatible porous scaffold is a tissue regeneration scaffold, preferably a bone regeneration scaffold.

4. Biocompatible porous scaffold according to the preceding claims, wherein at least one of the carbon-based nanoparticles, preferably diamond nanoparticles, is hydrophilic.

5. Biocompatible porous scaffold according to the preceding claims, wherein at least one of the carbon-based nanoparticles, preferably diamond nanoparticles, has an oxygen-terminated surface.

6. Biocompatible porous scaffold according to the preceding claims, wherein the carbon-based nanoparticles, preferably diamond nanoparticles, are not functionalized with biomolecules.

7. Biocompatible porous scaffold according to any one of claims 1 to 5, wherein at least one of the carbon-based nanoparticles, preferably diamond nanoparticles, is functionalized with a biomolecule bound to the at least one carbon-based nanoparticle, preferably diamond nanoparticle.

8. Biocompatible porous scaffold according to claim 7, wherein the biomolecule is bound to the at least one carbon-based nanoparticle, preferably diamond nanoparticle, via physisorption.

9. Biocompatible porous scaffold according to claim 7 or claim 8, wherein the biomolecule is present on the biocompatible porous scaffold as gradient.

10. Biocompatible porous scaffold according to the preceding claims, wherein at least two different kinds of biomolecules are bound to the at least one carbon-based nanoparticle.

11. Biocompatible porous scaffold according to the preceding claims, wherein the carbon-based nanoparticles are bound to at least a part of the surface of the biocompatible porous scaffold material.

12. Biocompatible porous scaffold according to any of claims 1 to 8, wherein the carbon-based nanoparticles are embedded into the biocompatible porous scaffold material.

13. Biocompatible porous scaffold according to the preceding claims, comprising at least two different biocompatible porous scaffold materials, wherein the carbon-based nanoparticles are bound to at least one of the two biocompatible porous scaffold materials.

14. Biocompatible porous scaffold according to the preceding claims, wherein the biocompatible porous scaffold comprises the carbon-based nanoparticles in an amount of at least 0,1 % by weight to at most 10 % by weight.

15. Biocompatible porous scaffold according to the preceding claims for the use of treating a tissue defect, preferably a bone defect.

16. Use of carbon-based nanoparticles, preferably diamond nanoparticles, for the production of a biocompatible porous scaffold.

17. Method for the production of a biocompatible porous scaffold according to any one of claims 1 to 15, wherein a biocompatible porous scaffold material is combined with carbon-based nanoparticles.

18. Method for the production of a biocompatible porous scaffold according to any one of claims 7 to 10, wherein biomolecules are bound to the at least one carbon-based nanoparticle, preferably diamond nanoparticle of a biocompatible porous scaffold according to any one of claims 1 to 6.

19. Kit of parts comprising at least one biocompatible porous scaffold according to any one of claims 1 to 6 and a manual, wherein the at least one biocompatible porous scaffold is sterilized.

20. Kit of parts comprising at least one biocompatible porous scaffold according to any one of claims 1 to 6 and at least one biomolecule, wherein the at least one biocompatible porous scaffold and the at least one biomolecule are physically separated in the kit of parts.
